(19) Europäisches Patentamt
European Patent Office
Office européen des brevets

(11) Veröffentlichungsnummer: **0 114 299**
**A2**

(12) # EUROPÄISCHE PATENTANMELDUNG

(21) Anmeldenummer: **83112573.7**

(22) Anmeldetag: **14.12.83**

(51) Int. Cl.³: **C 07 C 79/46**
C 07 C 76/02, A 01 N 37/10

(30) Priorität: **23.12.82 DE 3247669**

(43) Veröffentlichungstag der Anmeldung:
**01.08.84 Patentblatt 84/31**

(84) Benannte Vertragsstaaten:
**CH DE FR GB IT LI**

(71) Anmelder: **BASF Aktiengesellschaft**
**Carl-Bosch-Strasse 38**
**D-6700 Ludwigshafen(DE)**

(72) Erfinder: **Parg, Adolf, Dr.**
**Paray-le-Monial-Strasse 8**
**D-6702 Bad Duerkheim(DE)**

(72) Erfinder: **Hamprecht, Gerhard, Dr.**
**Rote-Turm-Strasse 28**
**D-6940 Weinheim(DE)**

(72) Erfinder: **Wuerzer, Bruno, Dr.**
**Ruedigerstrasse 13**
**D-6701 Otterstadt(DE)**

(54) **Phenylglyoxylsäurederivate, Verfahren zu ihrer Herstellung und ihre Verwendung zur Bekämpfung unerwünschten Pflanzenwuchses.**

(57) Die Erfindung betrifft Phenylglyoxylsäurederivate der Formel

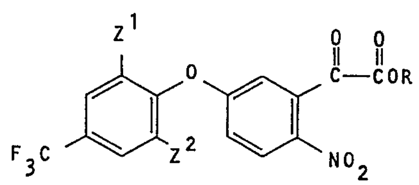

(I),

in der

$Z^1$ Wasserstoff oder Halogen,

$Z^2$ Halogen und

R Wasserstoff, Alkyl, einen gegebenenfalls substituierten araliphatischen Rest, einen gegebenenfalls substituierten Phenylrest, ein Alkalimetallion, ein Erdalkalimetallion oder ein gegebenenfalls substituiertes Ammoniumion bedeuten, Verfahren zur Herstellung dieser Verbindungen und ihre Verwendung zur Bekämpfung unerwünschten Pflanzenwuchses.

EP 0 114 299 A2

Phenylglyoxylsäurederivate, Verfahren zu ihrer Herstellung und ihre Verwendung zur Bekämpfung unerwünschten Pflanzenwuchses

Die Erfindung betrifft Phenylglyoxylsäurederivate, Verfahren zu ihrer Herstellung, Herbizide, die diese Verbindungen als Wirkstoffe enthalten, sowie Verfahren zur Bekämpfung unerwünschten Pflanzenwuchses mit diesen Wirkstoffen.

Es ist bekannt, phenoxysubstituierte Benzoesäurederivate, wie das Natriumsalz der 3-(2'-Chlor-4'-trifluormethylphenoxy)-6-nitro-benzoesäure oder deren Ester als Herbizide zu verwenden (DE-OS 23 11 638).

Es wurde gefunden, daß Phenylglyoxylsäurederivate der Formel

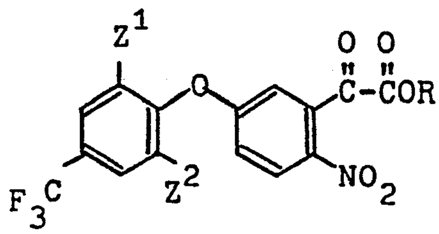

(I),

in der

$Z^1$ Wasserstoff oder Halogen,

$Z^2$ Halogen und

R Wasserstoff, $C_1$-$C_{20}$-Alkyl, einen gegebenenfalls substituierten araliphatischen Rest mit 7 bis 20 Kohlenstoffatomen, einen gegebenenfalls substituierten Phenylrest, ein Alkalimetallion, ein Äquivalent eines Erdalkalimetallions oder ein gegebenenfalls substituiertes Ammoniumion bedeuten, je nach Aufbereitung und Dosierung total oder selektiv herbizid wirksam sind.

In der Formel I bedeuten $Z^1$ Wasserstoff oder Halogen und $Z^2$ Halogen, wobei Halogen jeweils für Fluor, Chlor oder Brom steht.

R bedeutet Wasserstoff, $C_1$-$C_{20}$-Alkyl, vorzugsweise $C_1$-$C_8$-Alkyl, insbesondere $C_1$-$C_4$-Alkyl, beispielsweise Methyl, Ethyl, n-Propyl, i-Propyl, n-Butyl, i-Butyl, t-Butyl, isomere Pentylreste, isomere Hexylreste, isomere Heptylreste, isomere Octylreste, einen gegebenenfalls durch Halogen substituierten araliphatischen Rest mit 7 bis 20 Kohlenstoffatomen, vorzugsweise 7 bis 12 Kohlenstoffatomen, beispielsweise Benzyl, 4-Chlorbenzyl, 4-Fluorbenzyl, 2,4-Dichlorbenzyl, Phenethyl, 3-Phenyl-n-propyl, einen gegebenenfalls durch Halogen, wie Fluor, Chlor, Brom, $C_1$-$C_4$-Alkyl, wie Methyl, Ethyl, $C_1$-$C_4$-Halogenalkyl, wie Trifluormethyl, oder Cyano

H/Kl

substituierten Phenylrest, ein Alkalimetallion, wie Lithium, Kalium oder Natrium, ein Äquivalent eines Erdalkalimetallions, wie Magnesium, Calcium oder Barium, oder ein gegebenenfalls durch $C_1$-$C_4$-Alkyl substituiertes Ammoniumion, wie Ammonium, Alkylammonium, Dialkylammonium, Trialkylammonium, beispielsweise Methylammonium, Trimethylammonium, Ethylammonium, Diethylammonium, Triethylammonium oder n-Propylammonium.

Bevorzugte Phenylglyoxylsäurederivate sind Verbindungen der Formel I, in der $Z^1$ Wasserstoff, $Z^2$ Halogen, insbesondere Chlor oder Brom, und R Wasserstoff oder $C_1$-$C_8$-Alkyl, insbesondere $C_1$-$C_4$-Alkyl, bedeuten.

Die Phenylglyoxylsäurederivate der Formel I können nach folgenden Verfahren hergestellt werden:

Gemäß Verfahren a) nitriert man die Phenylglyoxylsäurederivate der Formel

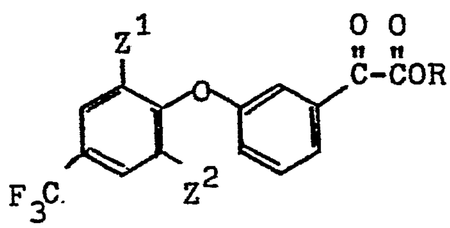 (II),

in der $Z^1$, $Z^2$ und R die obengenannten Bedeutungen haben, mit der äquivalenten Menge eines Nitriergemisches, bestehend aus konzentrierter Salpetersäure oder Alkalinitrat und konzentrierter Schwefelsäure, gegebenenfalls in Gegenwart eines organischen Lösungsmittels bei einer Temperatur im Bereich von -10 bis +25°C, drucklos oder unter Druck, kontinuierlich oder diskontinuierlich, zu den Verbindungen der Formel I.

Gemäß Verfahren b) setzt man das Phenolat der Formel

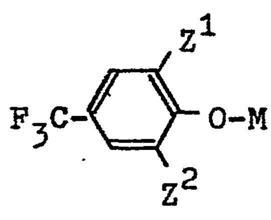 (III),

in der $Z^1$ und $Z^2$ die obengenannten Bedeutungen haben und M für ein Alkalimetallion, z.B. ein Natrium- oder Kaliumion, steht, mit einem Phenylglyoxylsäurederivat der Formel

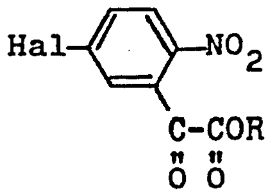

(IV),

in der R die obengenannten Bedeutungen hat und Hal für Halogen steht, in Gegenwart eines polaren, aprotischen Lösungsmittels bei einer Temperatur zwischen 40 und 220°C, drucklos oder unter Druck, kontinuierlich oder diskontinuierlich zu den Verbindungen der Formel I um.

Gemäß Verfahren c) verseift man das Acylcyanid der Formel

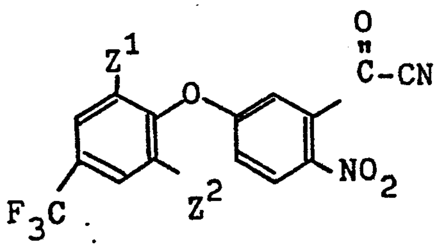

(V),

in der $Z^1$ und $Z^2$ die obengenannten Bedeutungen haben, mit Hilfe konzentrierter Mineralsäuren, z.B. Schwefelsäure, in Gegenwart von Alkoholen der Formel

R-OH (VI),

in der R die obengenannten Bedeutungen hat, gegebenenfalls in Gegenwart eines inerten organischen Lösungsmittels bei einer Temperatur zwischen 20 und 150 °C, drucklos oder unter Druck, kontinuierlich oder diskontinuierlich zu den Verbindungen der Formel I.

Das Verfahren zur Herstellung der Phenylglyoxylsäurederivate der Formel I nach Verfahrensweise a) läßt sich durch folgendes Formelschema wiedergeben:

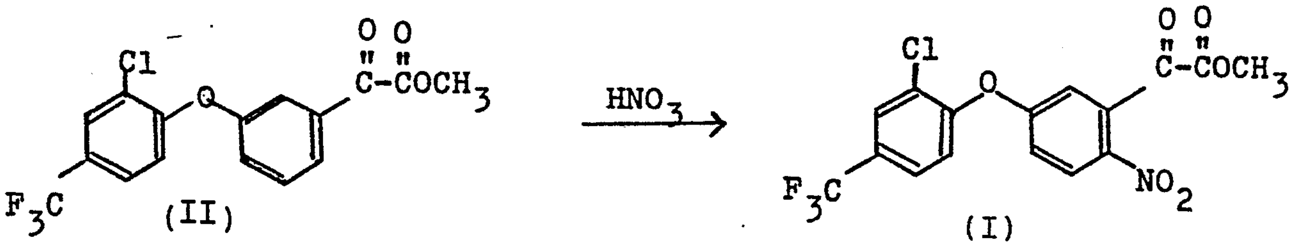


(II) (I)

Eine Lösung des Ausgangsstoffs II, beispielsweise in Essigsäure, Essigsäureanhydrid oder Dichlorethan, vorzugsweise in Essigsäureanhydrid, wird mit der stöchiometrischen Menge an konzentrierter Salpetersäure oder

Alkalinitrat, z.B. Kaliumnitrat, in Gegenwart der 1- bis 10-fachen molaren Menge an konzentrierter Schwefelsäure, bezogen auf das Nitrierungsmittel, bei Temperaturen von -10 bis +25°C, vorzugsweise 0 bis +15°C, innerhalb 0,5 bis 8 Stunden, insbesondere 1 bis 4 Stunden, nitriert. Zur Vervollständigung der Reaktion kann bei 25 bis 35°C über über 2 bis 8 Stunden nachgerührt werden. Zur Aufarbeitung rührt man das Reaktionsgemisch in Eis/Wasser ein und isoliert das Endprodukt durch Extraktion oder Absaugen; dieses kann durch Umfällen, Umkristallisieren oder gegebenenfalls durch Chromatographie gereinigt werden.

Das Verfahren b) läßt sich durch folgendes Formelschema wiedergeben:

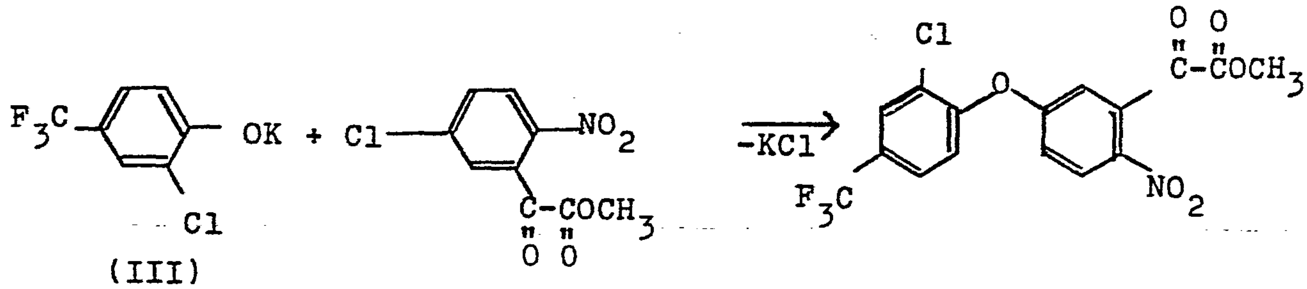


(III) (IV) (I)

Die Ausgangsstoffe III und IV werden in stöchiometrischer Menge in einem polaren, aprotischen Lösungsmittel gelöst und dann bei einer Temperatur von 40 bis 220°C, vorzugsweise 80 bis 160°C, innerhalb von 0,5 bis 48 Stunden, insbesondere 4 bis 30 Stunden, drucklos oder unter Druck, kontinuierlich oder diskontinuierlich umgesetzt. Zur Aufarbeitung rührt man das Reaktionsgemisch in Wasser ein und isoliert das Endprodukt durch Extraktion oder Absaugen; dieses kann durch Umfälllen, Umkristallisieren oder gegebenenfalls durch Chromatographie gereinigt werden.

Das Verfahren c) läßt sich durch folgendes Formelschema wiedergeben:

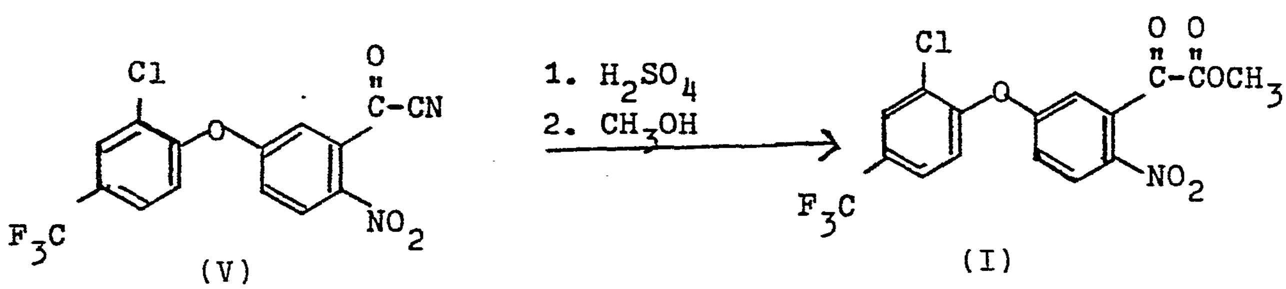


(V) (I)

Das Acylcyanid V wird gegebenenfalls in Gegenwart eines inerten organischen Lösungsmittels mit Hilfe der mindest stöchiometrischen Menge, vorzugsweise jedoch mit einem Überschuß von bis zu 100 Mol.%, bezogen auf Ausgangsstoff V, einer konzentrierten Mineralsäure, vorzugsweise Schwefelsäure, in Gegenwart der mindest stöchiometrischen Menge an Alkohol der

Formel VI bei einer Temperatur zwischen 20 und 150°C, vorzugsweise 30 bis 100°C, drucklos oder unter Druck, kontinuierlich oder diskontinuierlich umgesetzt. Zur Vervollständigung der Reaktion kann bei 30 bis 100°C über 2 bis 48 Stunden, vorzugsweise 4 bis 24 Stunden, nachgerührt werden. Zur Aufarbeitung rührt man das Reaktionsgemisch in Eis/Wasser ein und isoliert das Endprodukt durch Extraktion oder Absaugen; dieses kann durch Umfällen, Umkristallisieren oder gegebenenfalls durch Chromatographie gereinigt werden.

Im Verfahren b) verwendet man unter den jeweiligen Reaktionsbedingungen inerte polare, aprotische Lösungsmittel. Als Lösungsmittel kommen beispielsweise Sulfoxide, wie Dimethylsulfoxid, Diethylsulfoxid, Dimethylsulfon, Diethylsulfon, Methylethylsulfon oder Tetramethylensulfon, Amide, wie Dimethylformamid, Dimethylacetamid, N-Methylcaprolactam, N-Methylpyrrolidon, außerdem Tetramethylenharnstoff oder Hexamethylenphosphorsäuretriamid in Betracht.

Die Ausgangsverbindungen werden nach literaturbekannten Methoden hergestellt. So erhält man die Phenylglyoxylsäureester der Formel II und IV nach der in der DE-OS 27 081 89 beschriebenen Verfahrensweise. Acylcyanide der Formel V können nach den in Angew. Chemie 1982, S. 1 beschriebenen Verfahrensweisen hergestellt werden. Phenoxysubstituierte Benzoesäuren als Ausgangsprodukte für die Acylcyanide sind allgemein zugänglich oder können ebenfalls nach literaturbekannten Verfahren hergestellt werden (US-PS 4 031 131).

Das folgende Beispiel erläutert die Herstellung der Phenylglyoxylsäurederivate der Formel I nach Verfahrensweise a). Gewichtsteile verhalten sich zu Volumenteilen wie kg zu l.

Beispiel:

Eine Lösung von 32 Gewichtsteilen 3-(2'-Chlor-4'-trifluormethylphenoxy)--phenylglyoxylsäuremethylester in 80 Volumenteilen Essigsäureanhydrid wird innerhalb von 2 Stunden mit einer Mischung von 8 Volumenteilen konz. Salpetersäure und 3 Volumenteilen konz. Schwefelsäure bei einer Temperatur von 0 bis 5°C versetzt. Die Reaktionsmischung wird dann zwei Stunden bei Raumtemperatur nachgerührt und anschließend in Eis/Wasser eingerührt. Man saugt den Niederschlag ab, kristallisiert aus Ethanol um und erhält 25 Gewichtsteile (= 69 % der Theorie) 3-(2'-Chlor-4'-trifluormethylphenoxy)-6-nitro-phenylglyoxylsäuremethylester vom Schmelzpunkt 143 bis 145°C (Verbindung Nr. 1).

Entsprechend können beispielsweise die folgenden Verbindungen der Formel

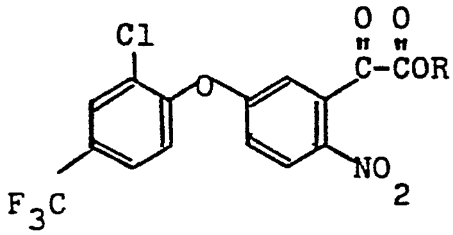

(I)

erhalten werden.

| Verbindung Nr. | R | Fp [°C]/$n_D^{25}$ |
|---|---|---|
| 1 | $CH_3$ | 143-145 |
| 2 | H | |
| 3 | Ethyl | 1,5408 |
| 4 | n-Propyl | 1,5291 |
| 5 | i-Propyl | 1,4884 |
| 6 | n-Butyl | 1,5112 |
| 7 | tert.-Butyl | 1,5482 |
| 8 | n-Pentyl | |
| 9 | n-Hexyl | |
| 10 | n-Heptyl | |
| 11 | Benzyl | |
| 12 | Phenethyl | |
| 13 | Phenyl | |
| 14 | 4-Chlorphenyl | |
| 15 | 2,4-Dichlorphenyl | |
| 16 | 3-Trifluormethylphenyl | |
| 17 | Tolyl | |
| 18 | $Na^+$ | |
| 19 | $K^+$ | |
| 20 | $NH_4^+$ | |

Die Phenylglyoxylsäurederivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden.

Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 10 Gewichtsteile der Verbindung Nr. 3 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 7 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 3 werden in einer Mischung gelöst, die aus 25 Gewichtsteilen Cyclohexanol, 65 Gewichtsteilen einer Mineralöl-fraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 6 werden mit 3 Gewichtsteilen des Natriumsalzes der Diisobutyl-naphthalin-sulfonsäure, 10 Gewichtsteilen des Na-triumsalzes einer Ligninsulfonsäure aus einer Sulfit--Ablauge und 7 Gewichtsteilen pulverförmigem Kieselsäuregel gut vermischt und in einer Hammermühle vermahlen.

VI. 5 Gewichtsteile der Verbindung Nr. 4 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 5 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadien, Bodenart und Anwendungsverfahren 0,005 bis 5, vorzugsweise 0,01 bis 3,0 kg/ha.

Die herbizide Wirkung der Phenylglyoxylsäurederivate der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät.

Bei Vorauflaufbehandlung werden die Wirkstoffe unmittelbar danach auf die Erdoberfläche aufgebracht. Sie werden hierzu in Wasser als Verteilungsmittel suspendiert oder emulgiert und mittels fein verteilender Düsen gespritzt. Bei dieser Applikationsmethode beträgt die Aufwandmenge 3,0 kg

Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,015 bis 0,06 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Amaranthus spp. (Fuchsschwanz), Chenopodium album (Weißer Gänsefuß), Echinochloa crus-galli (Hühnerhirse), Euphorbia heterophylla (Wolfsmilcharten), Hordeum vulgare (Gerste), Ipomoea spp. (Prunkwinde), Lamium amplexicaule (Stengelumfassende Taubnessel), Lolium multiflorum (Ital. Raygras), Oryza sativa (Reis), Sesbania exaltata (Turibaum), Sinapis alba (Weißer Senf), Solanum nigrum (Schwarzer Nachtschatten), Viola tricolor (Gewöhnliches Stiefmütterchen).

In diesen Versuchen zeigen beispielsweise die Wirkstoffe Nr. 1 und 3 bei Vorauflaufanwendung eine sehr gute Aktivität gegen die beispielhaft ausgewählten Testpflanzen.

Bei Nachauflaufanwendung wirken beispielsweise die Verbindungen Nr. 1 und 3 sehr stark gegen ein breites Spektrum unerwünschter Pflanzen. Die dabei an Kulturpflanzen auftretenden Blattverbrennungen sind unterschiedlich ausgeprägt und auf jeden Fall nur temporär; Kulturen, wie Getreide und Soja, regenerieren rasch durch nachwachsende Blätter.

In Anbetracht der Vielseitigkeit der Applikationsmethoden können die erfindungsgemäßen Verbindungen noch in einer weiteren Zahl von Kulturpflanzen zur Beseitigung von unerwünschtem Pflanzenwuchs, vorzugsweise breitblättriger annueller Arten, eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceium (Gossypium vitifolium) | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Hordeum vulgare | Gerste |
| Humulus lupulus | Hopfen |
| Ipomoea batatas | Süßkartoffeln |
| Juglans regia | Walnußbaum |
| Lacuta sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Tibes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |

| Botanischer Name | Deutscher Name |
|---|---|
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, Cyclohexan-1,3-dion-derivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

0114299

Patentansprüche

1. Phenylglyoxylsäurederivate der Formel

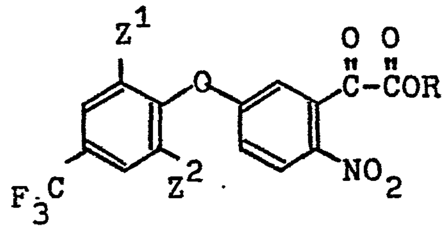

(I),

in der

$Z^1$ Wasserstoff oder Halogen,

$Z^2$ Halogen und

R Wasserstoff, $C_1$-$C_{20}$-Alkyl, einen gegebenenfalls durch Halogen substituierten araliphatischen Rest mit 7 bis 20 Kohlenstoffatomen, einen gegebenenfalls durch Halogen, $C_1$-$C_4$-Alkyl, $C_1$-$C_4$-Halogenalkyl oder Cyano substituierten Phenylrest, ein Alkalimetallion, ein Äquivalent eines Erdalkalimetallions oder ein gegebenenfalls substituiertes Ammoniumion bedeuten.

2. Phenylglyoxylsäurederivate der Formel I gemäß Anspruch 1, dadurch gekennzeichnet, daß $Z^1$ Wasserstoff, $Z^2$ Halogen und R Wasserstoff oder $C_1$-$C_8$-Alkyl bedeuten.

3. Phenylglyoxylsäurederivate der Formel I gemäß Anspruch 1, dadurch gekennzeichnet, daß $Z^1$ Wasserstoff, $Z^2$ Halogen und R Wasserstoff oder $C_1$-$C_4$-Alkyl bedeuten.

4. Verfahren zur Herstellung von Phenylglyoxylsäurederivaten der Formel I gemäß Anspruch 1, dadurch gekennzeichnet, daß man ein Phenylglyoxylsäurederivat der Formel

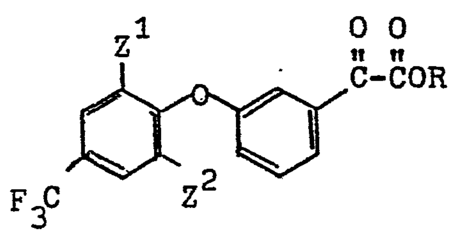

(II),

in der $Z^1$, $Z^2$ und R die im Anspruch 1 genannten Bedeutungen haben, mit der äquivalenten Menge eines Nitriergemisches, bestehend aus konzentrierter Salpetersäure oder Alkalinitrat und konzentrierter Schwefelsäure gegebenenfalls in Gegenwart eines organischen Lösungsmittels bei einer Temperatur im Bereich von -10 bis +25°C nitriert.

5. Verfahren zur Herstellung von Phenylglyoxylsäurederivaten der Formel I gemäß Anspruch 1, dadurch gekennzeichnet, daß man ein Phenolat der Formel

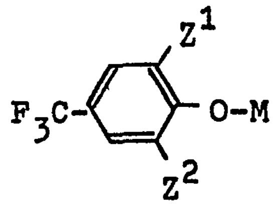 (III),

in der $Z^1$ und $Z^2$ die im Anspruch 1 genannten Bedeutungen haben und M für ein Alkalimetallion steht,
mit einem Phenylglyoxylsäurederivat der Formel

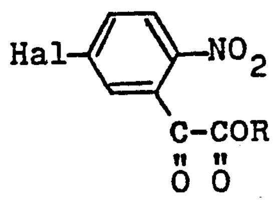 (IV),

in der R die im Anpruch 1 genannten Bedeutungen hat und Hal für Halogen steht,
in Gegenwart eines polaren, aprotischen Lösungsmittels bei einer Temperatur zwischen 40 und 220°C umsetzt.

6. Verfahren zur Herstellung von Phenylglyoxylsäurederivaten der Formel I gemäß Anspruch 1, dadurch gekennzeichnet, daß man ein Acylcyanid der Formel

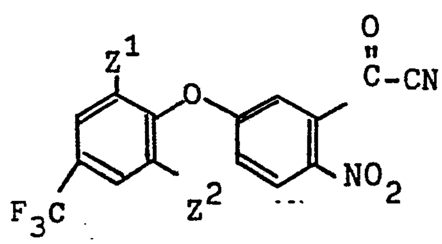 (V),

in der $Z^1$ und $Z^2$ die im Anspruch 1 genannten Bedeutungen haben,
mit Hilfe konzentrierter Mineralsäuren in Gegenwart eines Alkohols der Formel

R-OH (VI),

in der R die im Anspruch 1 genannten Bedeutungen hat, gegebenenfalls in Gegenwart eines inerten organischen Lösungsmittels bei einer Temperatur von 20 bis 150°C verseift.

7. Herbizid, enthaltend ein Phenylglyoxylsäurederivat der Formel I gemäß Anspruch 1.

8. Herbizid, enthaltend inerte Zusatzstoffe und ein Phenylglyoxylsäurederivat gemäß Anspruch 1.

9. Herbizid nach Anspruch 7, dadurch gekennzeichnet, daß es ein Phenylglyoxylsäurederivat der Formel I enthält, wobei $Z^1$ Wasserstoff, $Z^2$ Halogen und R Wasserstoff oder $C_1$-$C_8$-Alkyl bedeuten.

10. Verfahren zur Bekämpfung unerwünschten Pflanzenwuchses, dadurch gekennzeichnet, daß man die Pflanzen und/oder die von unerwünschtem Pflanzenwuchs freizuhaltende Fläche mit einer herbizid wirksamen Menge eines Phenylglyoxylsäurederivats der Formel I gemäß Anspruch 1 behandelt.